# FASCICULE DE BREVET EUROPEEN

(11) **EP 1 117 367 B1**
(45) Date de publication et mention de la délivrance du brevet: **11.02.2004**
(21) Numéro de dépôt: 99946277.3
(22) Date de dépôt: 01.10.1999
(51) Int. Cl.: A61K 6/093

(54) **COMPOSITION DENTAIRE A BASE D'UNE SILICONE RETICULABLE PAR VOIE CATIONIQUE**
DENTALHARZMASSE AUF BASIS VON KATIONISCH VERNETZBAREN POLYSILOXANEN
DENTAL COMPOSITION BASED ON SILICONE CROSSLINKABLE BY CATIONIC PROCESS

(30) Priorité: 02.10.1998 FR 9812374
(43) Date de publication de la demande: 25.07.2001
(73) Titulaire: RHODIA CHIMIE, 92408 Courbevoie Cédex (FR)
(72) Inventeur: FRANCES, Jean-Marc, F-69330 Meyzieu (FR)
(86) Numéro de dépôt international: PCT/FR1999/002344
(87) Numéro de publication internationale: WO 2000/019966

(56) Documents cités:
- EP-A- 0 562 897
- EP-A- 0 867 443
- WO-A-92/16183
- GB-A- 2 086 914

## Description

Le domaine de l'invention est celui des compositions dentaires. Plus précisément, les compositions dentaires mises au point dans le cadre de la présente invention sont utilisables pour la réalisation de prothèses dentaires et pour la restauration dentaire.

A ce jour, pour réaliser des compositions dentaires pour la préparation de prothèses dentaires ou de matériaux de restauration dentaire, on peut utiliser des résines à base d'acrylates photopolymérisables. Ces produits *prêt-à-formuler* présentent toutefois à l'utilisation des problèmes d'irritation et des problèmes potentiels de toxicité.

En outre, ces produits présentent l'inconvénient majeur d'engendrer un retrait volumique important lors de leur polymérisation: ce qui rend leur utilisation complexe et difficile pour la réalisation de prothèses dentaires ou de matériaux de restauration dentaire. On observe notamment des problèmes d'accrochage dus au retrait volumique ou au manque d'adhérence des polymères utilisés.

La présente invention a pour objet de fournir de nouvelles compositions dentaires ne présentant pas les inconvénients de l'art antérieur. Ces nouvelles compositions dentaires, polymérisables et/ou réticulables en environnement oral, ont des qualités nettement améliorées, notamment en ce qui concerne la réduction très nette du phénomène de retrait des compositions dentaires utilisées pour la. réalisation de prothèses dentaires ou de matériaux de restauration dentaire.

La composition dentaire polymérisable et/ou réticulable selon l'invention comprend :
**(1)** au moins un oligomère ou polymère silicone réticulable et/ou polymérisable, liquide à température ambiante ou thermofusible à température inférieure à 100°C, et comprenant :
   - au moins un motif de formule (I) : 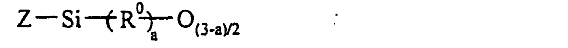 dans laquelle :
      - a = 0, 1 ou 2,
      - R⁰ , identique ou différent, représente un radical alkyle, cycloalkyle, aryle, vinyle, hydrogéno, alcoxy, de préférence un alkyle inférieur en C₁-C₆,
      - Z, identique ou différent, est un substituant organique comportant au moins une fonction réactive époxy, et/ou alcénylether et/ou oxétane et/ou dioxolane et/ou carbonate, et de préférence Z étant un substituant organique comportant au moins une fonction réactive époxy et/ou dioxolane,
   - et au moins deux atomes de silicium ;
**(2)** une quantité efficace d'au moins un photoamorceur de type borate de complexe organométallique, ayant une absorption résiduelle de la lumière comprise entre 200 et 500 nm ;
**(3)** et au moins une charge dentaire présente dans une proportion d'au moins 10% en poids par rapport au poids total de la composition.

Selon une première variante de la présente invention, la composition dentaire est polymérisable et/ou réticulable sous activation par voie thermique ou par voie photochimique.

En général, l'activation photochimique est réalisée sous rayonnement U.V. Plus particulièrement, on utilise un rayonnement U.V. de longueur d'onde de l'ordre de 200 à 500 nm pour la réalisation de prothèses dentaires et un rayonnement U.V. visible de longueur d'onde supérieur à 400 nm pour la réalisation de matériaux de restauration. Une longueur d'onde supérieure à 400 nm permet la réticulation et/ou polymérisation en environnement oral.

Le polymère ou oligomère silicone (1) présente l'avantage par rapport à des résines organiques d'être transparent à la lumière U.V.-visible et donc son utilisation permet d'obtenir des matériaux très épais et dont la photoréticulation s'effectue en peu de temps.

Les fonctions réactives Z du polymère ou oligomère silicone (1) peuvent être très variées. Toutefois, des compositions dentaires particulièrement intéressantes sont obtenues lorsque l'oligomère ou polymère silicone (1) comprend au moins un motif (FS) dans lequel Z représente un substituant organique Z1 comportant au moins une fonction réactive époxy, et/ou dioxolane, et de préférence au moins une fonction réactive époxy.

Selon deux alternatives avantageuses de la présente invention, l'oligomère ou polymère silicone (1) avec au moins une fonction réactive Z1 époxy, et/ou dioxolane, et de préférence au moins une fonction réactive époxy peut :
(i) soit comporter uniquement ce(s) type(s) de fonction(s) réactive(s) Z1,
(ii) ou soit comporter d'autres fonctions réactives Z telles que les fonctions réactives Z2 alcénylether, oxétane et/ou carbonate.

Dans le cas de la première alternative (i), la composition dentaire peut également comprendre d'autres oligomères et/ou polymères silicones comportant d'autres fonctions réactives Z2 telles que les fonctions alcénylether, oxétane et/ou carbonate et éventuellement des fonctions réactives Z1.

A titre d'exemples de fonctions réactives Z, celles-ci peuvent être notamment choisies parmi les radicaux suivants :

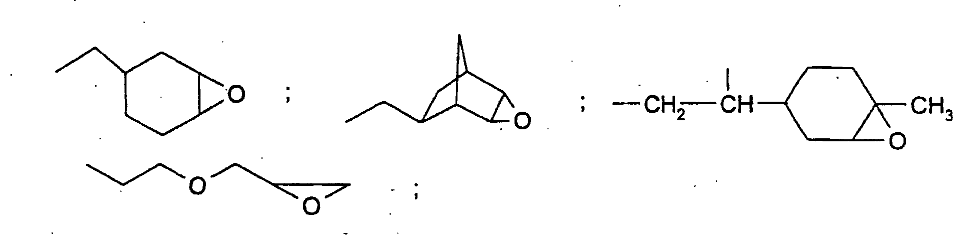

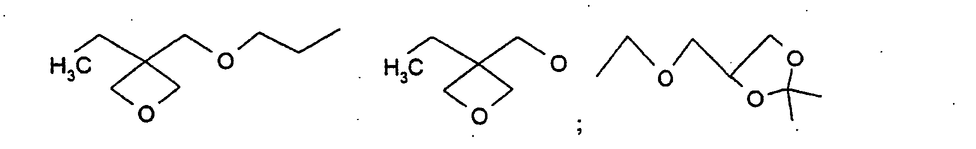

―(CH₂)₃―O―CH=CH₂ ;

―(CH₂)₃―O―CH=CH― R"

- avec R" représentant un radical alkyle linéaire ou ramifié en C₁-C₆.

Selon une variante avantageuse de la présente invention, le polymère ou oligomère silicone est constitué par au moins une silicone de formule moyenne suivante:

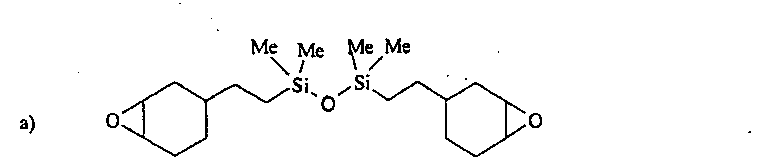

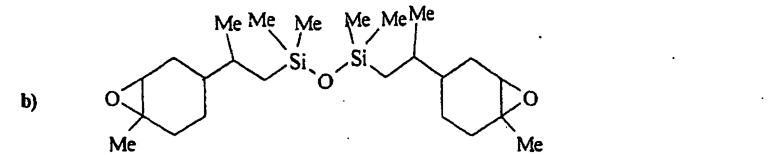

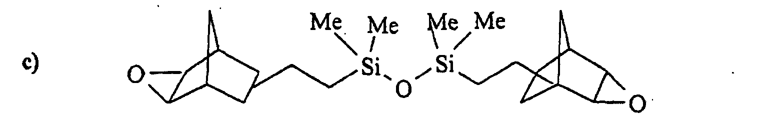

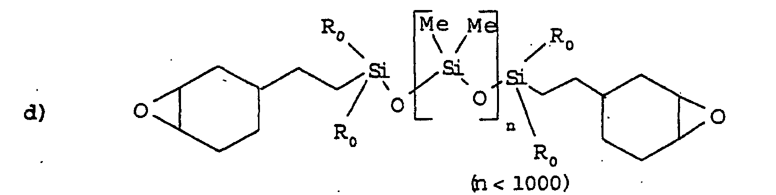

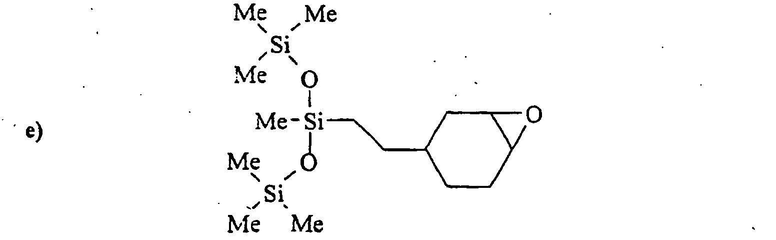

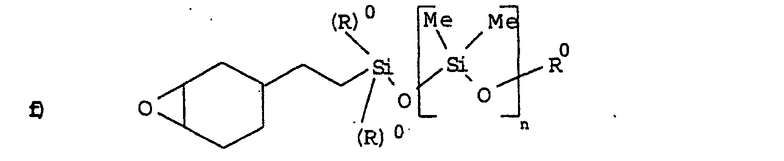

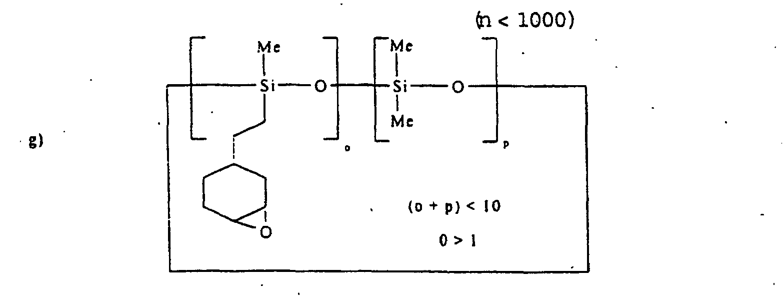

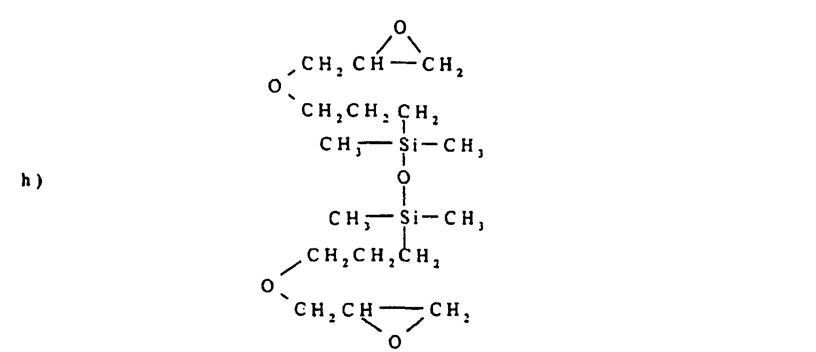

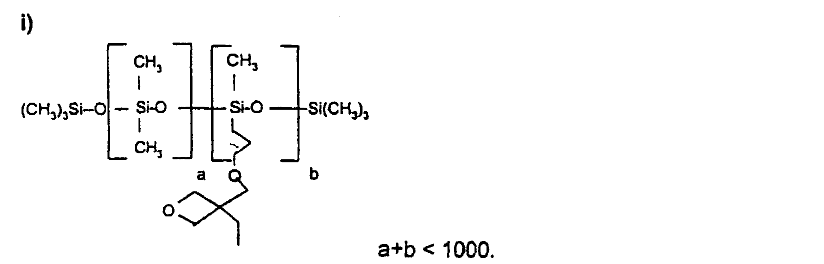

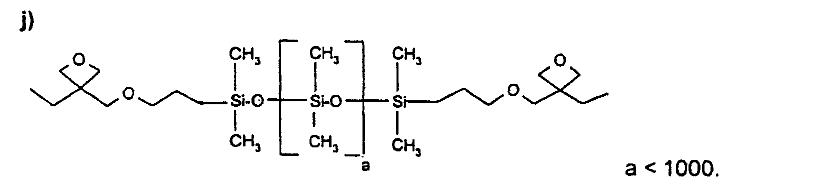

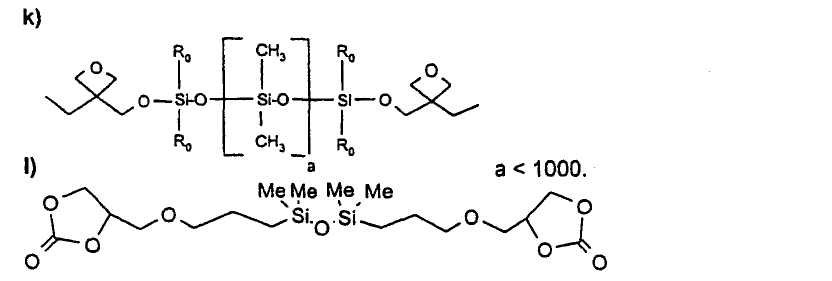

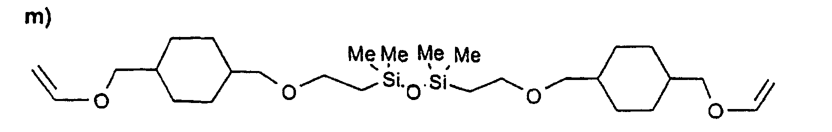

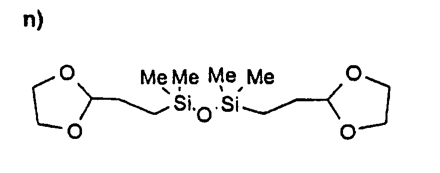

Les photoamorceurs cationiques peuvent être choisis parmi les borates d'un complexe organométallique (pris à eux seuls ou en mélange entre eux) d'un élément des groupes 4 à 10 de la classification périodique [Chem. & Eng. News, vol.63, N° 5, 26 du 4 février 1985].

L'entité cationique du borate est sélectionnée parmi les sels organométalliques de formule (II) :

(L¹L²L³M)^{+q}

formule dans laquelle :
- M représente un métal du groupe 4 à 10, notamment du fer, manganèse, chrome, cobalt,
- L¹ représente 1 ligand lié au métal M par des électrons π, ligand choisi parmi les ligands η³-alkyl, η⁵⁻ cyclopendadiènyl et η⁷⁻ cycloheptratriènyl et les composés η⁶ - aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons π ;
- L² représente un ligand lié au métal M par des électrons π, ligand choisi parmi les ligands η⁷-cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π ;
- L³ représente de 0 à 3 ligands identiques ou différents liés au métal M par des électrons σ, ligand(s) choisi(s) parmi CO et NO₂⁺ ; la charge électronique totale q du complexe à laquelle contribuent L¹, L² et L³ et la charge ionique du métal M étant positive et égale à 1 ou 2 ;

L'entité anionique borate a pour formule [BXₐ R_{b}]⁻ (III) dans laquelle :
- a et b sont des nombres entiers allant pour a de 0 à 3 et pour b de 1 à 4 avec a + b = 4,
- les symboles X représentent :
   * un atome d'halogène (chlore, fluor) avec a = 0 à 3,
   * une fonction OH avec a = 0 à 2,
- les symboles R sont identiques ou différents et représentent :
   ▷ un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
   ▷ un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10
   ▷ un radical aryle contenant au moins deux noyaux aromatiques tel que par exemple biphényle, naphtyle, éventuellement substitué par au moins un élément ou un groupement électroattracteur, notamment un atome d'halogène (fluor tout particulièrement), OCF₃, CF₃, NO₂, CN, quelle que soit l'entité cationique.

Dans le cadre de la présente invention, les photoamorceurs utilisés sont sélectionnés avec une absorption résiduelle de la lumière U.V. comprise entre 200 et 500 nm, de préférence 400 à 500 nm pour les préparations de prothèses dentaires. Pour la restauration dentaire, on préférera un photoamorceur ayant une absorption résiduelle de la lumière U.V. au-delà de 400 nm.

Sans que cela ne soit limitatif, on donne ci-après plus de précisions quant aux sous classes de borate de sels organométalliques plus particulièrement préférés dans le cadre des utilisations conformes à l'invention.

Selon une première variante préférée de l'invention, les espèces de l'entité anionique borate qui conviennent tout particulièrement sont les suivantes :

| | | | |
|---|---|---|---|
| **1'** | [B(C₆F₅)₄]⁻ | **5'** | [B(C₆H₃(CF₃)₂)₄]⁻ |
| **2'** | [(C₆F₅)₂BF₂]⁻ | **6'** | [B (C₆H₃F₂)₄]⁻ |
| **3'** | [B(C₆H₄CF₃)₄]⁻ | **7'** | [C₆F₅BF₃]⁻ |
| **4'** | [B(C₆F₄OCF₃)₄]⁻. | | |

Selon une seconde variante préférée, les sels organométalliques (4) utilisables sont décrits dans les documents US-A-4 973 722, US-A-4 992 572, EP-A-203 829, EP-A-323 584 et EP-A-354 181. Les sels organométalliques plus volontiers retenus selon l'invention sont notamment :
. le (η⁵ - cyclopentadiènyle) (η⁶ - toluène) Fe⁺,
. le (η⁵ - cyclopentadiènyle) (η⁶ - méthyl-1-naphtalène) Fe⁺,
. le (η⁵ - cyclopentadiènyle) (η⁶ - cumène) Fe⁺,
. le bis (η⁶ - mesitylène) Fe⁺, le bis (η⁶ - benzène) Cr⁺.

En accord avec ces deux variantes préférées, on peut citer, à titre d'exemples de photoamorceurs du type borates d'onium, les produits suivants :
. (η⁵ - cyclopentadiènyle) (η⁶ - toluène) Fe⁺, [B(C₆F₅)₄]⁻
. (η⁵ - cyclopentadiènyle) (η⁶ - méthyl1-naphtalène) Fe⁺, [B(C₆F₅)₄]⁻
. (η⁵ - cyclopentadiènyle) (η⁶ - cumène) Fe⁺, [B(C₆F₅)₄]⁻

Comme autre référence littéraire pour définir les borates de sels organométalliques (4), on peut citer l'ensemble du contenu des demandes de brevet EP 0 562 897 et 0 562 922. Ce contenu est intégralement incorporé par référence dans le présent exposé.

Outre les trois principaux composants de la composition dentaire, celle ci peut comprendre au moins un photosensibilisateur hydrocarboné aromatique à un ou plusieurs noyaux aromatiques substitués ou non, ayant une absorption résiduelle de la lumière comprise entre 200 et 500 nm.

Ce photosensibilisateur peut être de nature très variée. Celui-ci peut répondre notamment à l'une des formule **(IV)** à **(XXII)** suivantes : 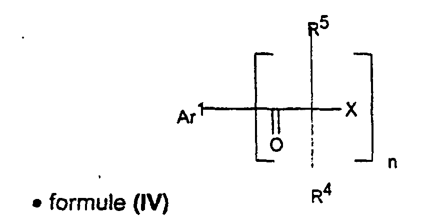 dans laquelle :
- lorsque n = 1, **Ar**^{**1**} représente un radical aryle contenant de 6 à 18 atomes de carbone, un radical tétrahydronaphtyle, thiényle, pyridyle ou furyle ou un radical phényle porteur d'un ou plusieurs substituants choisis dans le groupe constitué de F, Cl, Br, CN, OH, les alkyles linéaires ou ramifiés en C₁-C₁₂, -CF³, -OR⁶, -OPhényle, -SR⁶, -SPhényle, -SO₂Phényle, -COOR⁶, -O-(CH₂-CH=CH₂), -O(CH₂H₄-O)ₘ-H, -O(C₃H₆O)ₘ-H, m étant compris entre 1 et 100,
- lorsque n = 2, **Ar**_{**1**} représente un radical arylène en C₆-C₁₂ ou un radical phénylène-T-phénylène, où T représente -O-, -S-, -SO₂- ou -CH₂-,
- **X** représente un groupe -OR⁷ ou -OSiR⁸(R⁹)₂ ou forme, avec R⁴, un groupe -O-CH(R¹⁰)-,
- **R**_{**4**} représente un radical alkyle linéaire ou ramifié en C₁-C₈ non substitué ou porteur d'un groupe -OH, -OR⁶, acyloxy en C₂-C₈, -COOR⁶, -CF³, ou - CN, un radical alcényle en C₃ ou C₄, un radical aryle en C₆ à C₁₈, un radical phénylalkyle en C₇ à C₉,
- **R**^{**5**} a l'une des significations données pour R⁴ ou représente un radical -CH₂CH₂R¹¹, ou encore forme avec R⁴, un radical alkylène en C₂-C₈ ou un radical oxa-alkylène ou aza-alkylène en C₃-C₉,
- **R**^{**6**} représente un radical alkyle inférieur contenant de 1 à 12 atomes de carbone,
- **R**^{**7**} représente un atome d'hydrogène, un radical alkyle en C₁-C₁₂, un radical alkyle en C₂-C₆ porteur d'un groupe -OH, -OR⁶ ou -CN, un radical alcényle en C₃-C₆, un radical cyclohexyle ou benzyle, un radical phényle éventuellement substitué par un atome de chlore ou un radical alkyle linéaire ou ramifié en C₁-C₁₂, ou un radical tétrahydropyrannyle-2,
- **R**^{**8**} et **R**^{**9**} sont identiques ou différents et représentent chacun un radical alkyle en C₁-C₄ ou un radical phényle,
- **R**^{**10**} représente un atome d'hydrogène, un radical alkyle en C₁-C₈ ou un radical phényle,
- **R**^{**11**} représente un radical -CONH₂, -CONHR⁶, -CON(R⁶)₂, -P(O)(OR⁶)₂ ou pyridyle-2 ; 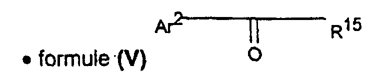 dans laquelle :
- Ar² a la même signification que Ar¹ de la formule **(IV)** dans le cas où n = 1,
- **R**^{**15**} représente un radical choisi parmi le groupe constitué d'un radical **Ar**^{**2**}**,** un radical -(C=O)-**Ar**^{**2**}, un radical alkyle linéaire ou ramifié en C₁-C₁₂, un radical cycloalkyle en C₆-C₁₂, et un radical cycloalkyle formant un cycle en C₆-C₁₂ avec le carbone de la cétone ou un carbone du radical **Ar**^{**2**}**,** ces radicaux pouvant être substitués par un ou plusieurs substituants choisis dans le groupe constitué de -F, -Cl, -Br, -CN, -OH, -CF₃,, -OR⁶, -SR⁶, -COOR⁶, les radicaux alkyles linéaires ou ramifiés en C₁-C₁₂ porteurs éventuellement d'un groupe -OH, -OR⁶ et/ou -CN, et les radicaux alcényles linéaires ou ramifiés en C₁-C₈ ; 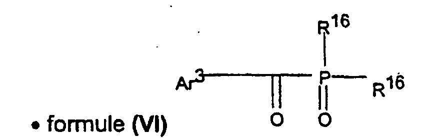
dans laquelle :
- **Ar**^{**3**} a la même signification que Ar¹ de la formule **(IV)** dans le cas où n = 1,
- **R**^{**16**}**,** identique ou différent, représente un radical choisi parmi le groupe constitué d'un radical Ar³, un radical -(C=O)-Ar³, un radical alkyle linéaire ou ramifié en C₁-C₁₂, un radical cycloalkyle en C₆-C₁₂, ces radicaux pouvant être substitués par un ou plusieurs substituants choisis dans le groupe constitué de -F, -Cl, -Br, -CN, -OH, -CF₃, -OR⁶, -SR⁶, -COOR⁶, les radicaux alkyles linéaires ou ramifiés en C₁-C₁₂ porteurs éventuellement d'un groupe - OH, -OR⁶ et/ou -CN, et les radicaux alcényles linéaires ou ramifiés en C₁-C₈; 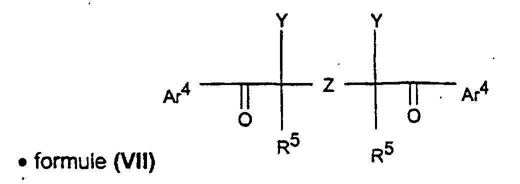
dans laquelle :
- **R**^{**5**}**,** identiques ou différents, ont les mêmes significations que dans la formule **(III),**
- **Y**, identiques ou différents, représentent X et/ou R⁴,
- **Z** représente :
   · une liaison directe,
   · un radical divalent alkylène en C₁-C₆, ou un radical phénylène, diphénylène ou phénylène-T-phénylène, ou encore forme, avec les deux substituants R⁵ et les deux atomes de carbone porteurs de ces substituants, un noyau de cyclopentane ou de cyclohexane,
   · un groupe divalent -O-R¹²-O-, -O-SiR⁸R⁹-O-SiR⁸R⁹-O-, ou -O-SiR⁸R⁹-O-,
- **R**^{**12**} représente un radical afkylène en C₂-C₈, alcénylène en C₄-C₆ ou xylylène.
   et **Ar**^{**4**} a la même signification que Ar¹ de la formule **(IV)** dans le cas où n = 1.
   - famille des thioxanthones de formule **(VIII)** : 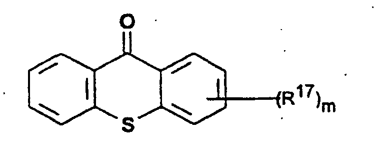
- m = 0 à 8,
- **R**^{**17**}**,** identique(s) ou différent(s) substituants sur le(s) noyau(x) aromatique(s), représentent un radical alkyle linéaire ou ramifié en C1-C12, un radical cycloalkyle en C6-C12, un radical Ar¹, un atome d'halogène, un groupement -OH, -CN, -NO₂, -COOR⁶, -CHO, Ophényle, -CF₃, -SR⁶, -Sphényle , -SO₂ phényle , Oalcényle ,ou -SiR⁶₃.
   - famille des xanthènes de formule **(IX)** : 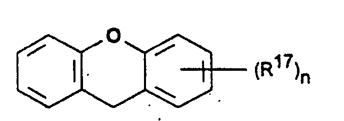 n = 0 à 8
   - famille des xanthones de formule **(X)**: 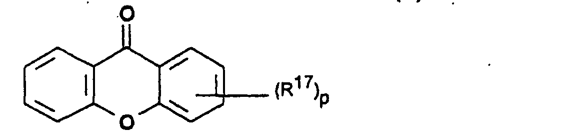 p = 0 à 8
   - famille du naphtalène de formule **(XI)**: 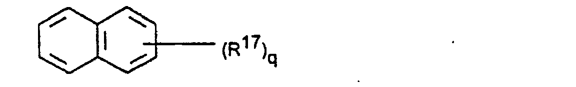 q= 0 à 8
   - famille de l'anthracène de formule **(XII)** : 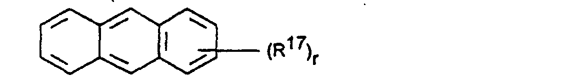 r = 0 à 10
   - famille du phénanthrène de formule **(XIII)** : 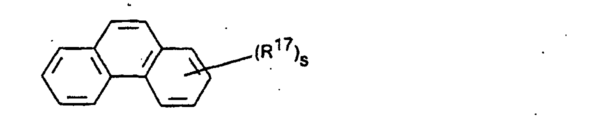 s = 0 à 10
   - famille du pyrène de formule **(XIV)** : 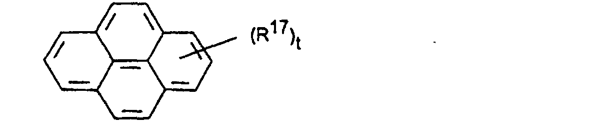 t = 0 à 10
   - famille du fluorène de formule **(XV)** : 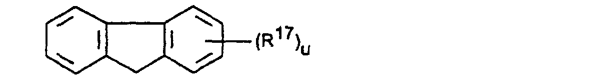 u = 0 à 9
   - famille du fluoranthène de formule (XVI): 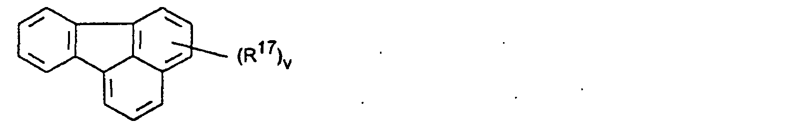 v = 0 à 10
   - famille du chrysène de formule **(XVII)** : 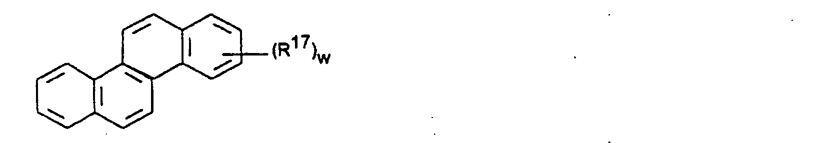 w = 0 à 12
   - famille de la fluorène de formule **(XVIII)** : 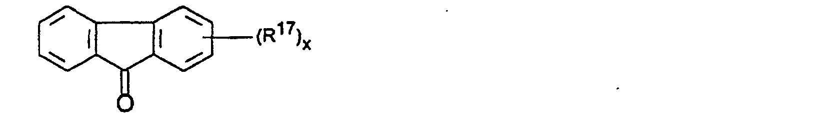 avec x = 0 à 8, par exemple 2,7 dinitro9-fluorénone,
   - famille de la chromone de formule **(XIX)** : 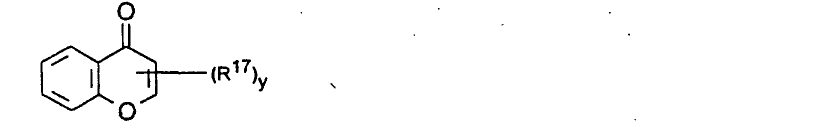 avec y = 0 à 6
   - famille de l'éosine de formule **(XX)** : 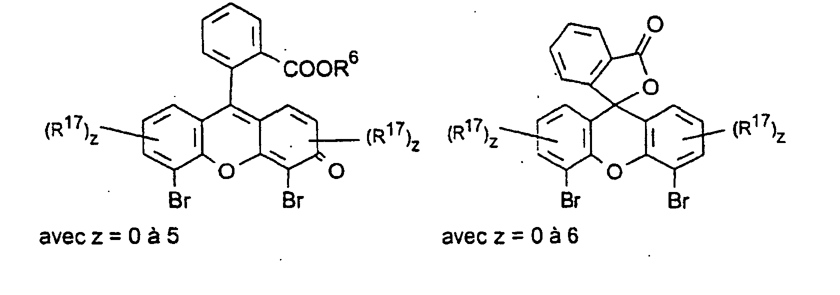
   - famille de l'érythrosine de formule **(XXI)** : 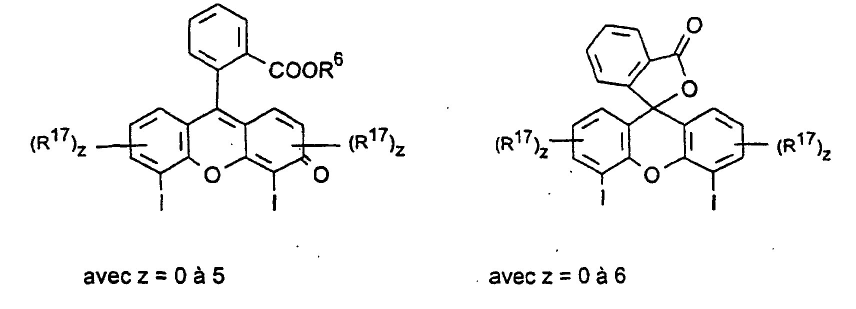
   - famille des biscoumarins de formule **(XXII)** : 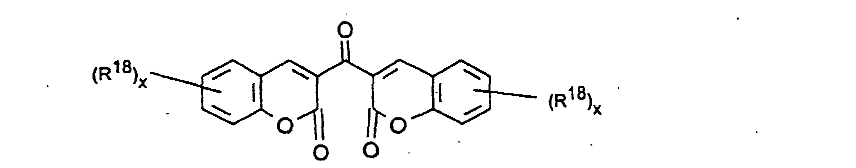
- **R**^{**18**}**,** identique ou différent, a la même signification que **R**^{**17**} ou représente un groupement -NR⁶₂, par exemple le 3,3'carbonylbis(7-diéthylaminocoumarin) et le 3,3'-carbonylbis(7-méthoxycoumarin).

D'autres sensibilisateurs sont utilisables. Notamment, on peut utiliser les photosensibilisateurs décrits dans les documents US 4,939,069; US 4,278,751; US 4,147,552.

Dans le cadre de la présente invention, comme pour les photoamorceurs, les photosensiblisateurs ont une absorption résiduelle de la lumière U.V. comprise entre 200 et 500 nm, de préférence 400 à 500 nm pour les préparations de prothèses dentaires. Pour la restauration dentaire, on préférera un photosensibilisateur ayant une absorption résiduelle de la lumière U.V. au-delà de 400 nm.

Selon une variante préférée, les photosensibilisateurs seront choisis parmi ceux des familles **(IV), (VII)** et **(VIII).** A titre d'exemples, on citera les phosensibilisateurs suivants:

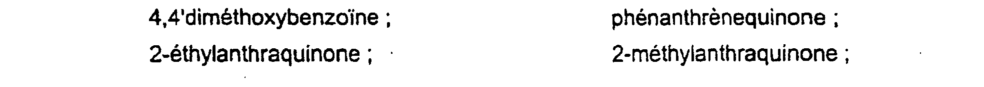

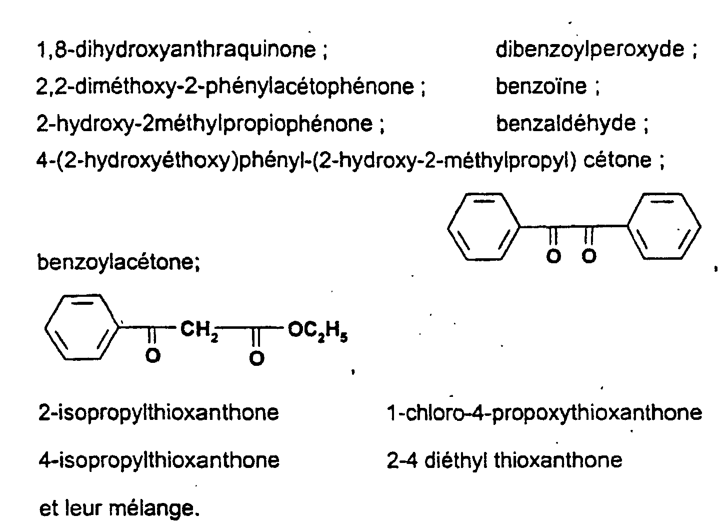

Différents types de charges sont utilisables pour préparer les compositions selon l'invention. Les charges sont choisies en fonction de l'utilisation finale de la composition dentaire : celles-ci affectent d'importantes propriétés telles que l'apparence, la pénétration du rayonnement U.V., ainsi que les propriétés mécaniques et physiques du matériau obtenu après réticulation et/ou polymérisation de la composition dentaire.

Comme charge de renforcement, on peut utiliser des charges de silice de pyrogénation traitée ou non, des charges de silice amorphe, du quartz, des verres ou des charges non vitreuses à base d'oxydes de zirconium, de baryum, de calcium, de fluor, d'aluminium, de titane, de zinc, des borosilicates, des aluminosilicates, du talc, des sphérosil, du trifluorure d'yterbium, des charges à base de polymères sous forme de poudre broyée tels que des polyméthacrylates de méthyle inertes ou fonctionnalisés, des polyépoxydes ou des polycarbonates.

A titre d'exemple, on citera :
- des charges inertes à base de polyméthacrylate de méthyle LUXASELF de la société UGL utilisables dans le domaine dentaire et pigmentées en rose,
- des charges de silice de combustion traitée hexaméthyldisilazane de surface spécifique 200 m²/g,
- des charges de silice de combustion non traitée (produit Aerosil AE200 commercialisée par DEGUSSA ).

Selon une variante avantageuse de l'invention , les charges et en particulier les charges de silice, sont traitées avant utilisation à 120 °C avec une quantité inférieure à 10% p/p de silicone comprenant au moins un motif de formule **(XXIII)** : 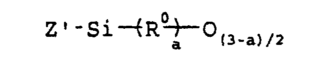
- tel que Z' a la même définition que Z
- a= 0,1 ,2 ou 3
- avec au moins un atome de silicium.

On peut citer à titre d'exemple, le polymère décrit ci-dessous avec Z= époxyde et Z= trialcoxysilyle :

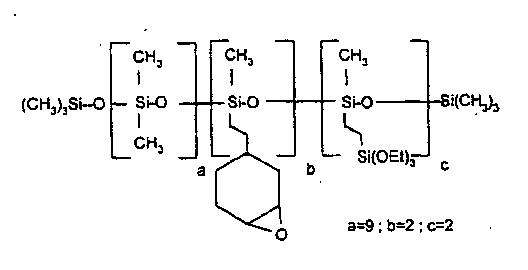

Dans ce cas de traitement de ou des charges siliciées en particulier la silice avec ce type de polymère, le matériau obtenu après réticulation présente une tenue mécanique, un module d'élasticité, et une résistance à la compression nettement améliorés.

Outre les charges de renforcement, des pigments peuvent être utilisés pour teinter la composition dentaire selon l'utilisation envisagée et les groupes ethniques.

Par exemple, on utilise des pigments rouges en présence de microfibres pour les compositions dentaires utilisées pour la préparation de prothèses dentaires afin de simuler les vaisseaux sanguins.

On emploie aussi des pigments à base d'oxydes métalliques (oxydes de fer et/ou titane et/ou aluminium et/ou zirconium, etc.) pour les compositions dentaires utilisées pour la préparation de matériau de restauration, afin d'obtenir un matériau réticulé de couleur ivoire.

D'autres additifs peuvent être incorporés au sein des compositions dentaires selon l'invention. Par exemple, des biocides, des stabilisants, des agents de flaveur, des plastifiants et des promoteurs d'adhérence.

Parmi les additifs envisageables, on utilisera avantageusement des co-réactifs réticulables et/ou polymérisables de type organique. Ces co-réactifs sont liquides à température ambiante ou thermofusibles à température inférieure à 100 °C, et chaque co-réactif comprend au moins deux fonctions réactives tels que oxétane-alcoxy, oxétane-hydroxy, oxétane-alcoxysilyle, carboxy-oxétane, oxétane-oxétane, alcénylether-hydroxy, alcénylether-alcoxysilyle, époxy-alcoxy, époxy-alcoxysilyles, dioxolane-dioxolane- alcool, etc.

Les compositions dentaires selon l'invention peuvent être utilisées pour de nombreuses applications dentaires, et en particulier dans le domaine des prothèses dentaires, dans le domaine de la restauration dentaire et dans le domaine des dents provisoires.

La composition dentaire selon l'invention se présente de préférence sous la forme d'un seul produit contenant les différents composants ("*monocomposant*") ce qui facilite sa mise en oeuvre, notamment dans le domaine des prothèses dentaires. Eventuellement, la stabilité de ce produit "monocomposant" peut être assurée par des dérivés organiques à fonctions amines selon l'enseignement du document WO 98/07798.

Sous la forme "monocomposant", le produit peut être par exemple déposé à l'aide d'une seringue directement sur le modèle en plâtre ou dans une clé. Puis, il est polymérisé (polymérisation par couches successives possibles) à l'aide d'une lampe U.V. (spectre lumière visible 200 - 500 nm). En général, la réalisation d'une prothèse dentaire esthétique et durable s'effectue en 10 à 15 mn.

Il est à noter que les produits obtenus à partir de la composition dentaire selon l'invention sont non poreux. Ainsi, après un éventuel polissage à l'aide d'une brosse feutre par exemple, la surface des prothèses dentaires obtenues est lisse et brillante et donc ne nécessite pas d'utilisation de vernis.

Les applications dans le domaine des prothèses dentaires sont essentiellement celles de la prothèse adjointe, que l'on peut diviser en deux types :
- prothèse totale en cas de patient complètement édenté,
- prothèse partielle due à l'absence de plusieurs dents se traduisant par soit une prothèse provisoire, soit un appareil squeletté.

Dans le domaine de la restauration dentaire, la composition dentaire selon l'invention peut être utilisée en tant que matériau d'obturation des dents antérieures et postérieures en différentes teintes (par exemple, teintes 'VITA"), rapide et facile à mettre en oeuvre.

La composition dentaire étant non toxique et polymérisable en couches épaisses, il n'est pas indispensable de polymériser le matériau en couches successives. En général, une seule injection de la composition dentaire est suffisante.

Les préparations pour prothèses dentaires et pour matériaux de restauration sont effectuées selon les techniques usuelles du métier.

Dans le cas d'application de la composition dentaire à une dent, soit la dent peut être pré-traitée avec un primaire d'accrochage ou soit la composition dentaire peut être préparée en mélange avec un primaire d'accrochage avant son utilisation. Toutefois, il n'est pas indispensable d'utiliser un primaire d'accrochage pour utiliser la composition dentaire selon l'invention.

Les exemples et tests suivants sont donnés à titre illustratif. Ils permettent notamment de mieux comprendre l'invention et de faire ressortir certains de ces avantages et d'illustrer quelques unes de ses variantes de réalisation.

### Exemples et Tests.

Les produits utilisés dans les compositions des exemples sont les suivants : 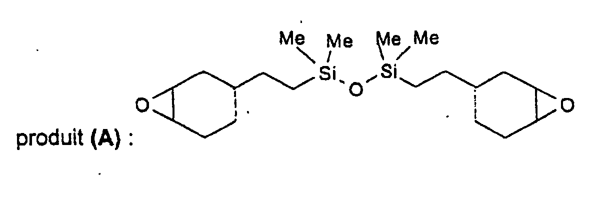 produit **(B)** : ce produit est un mélange des siioxanes, dont la viscosité est de 23,5 mPa.s et dont les proportions en poids et formules B₁, B₂ et B₃ sont données ci-dessous : 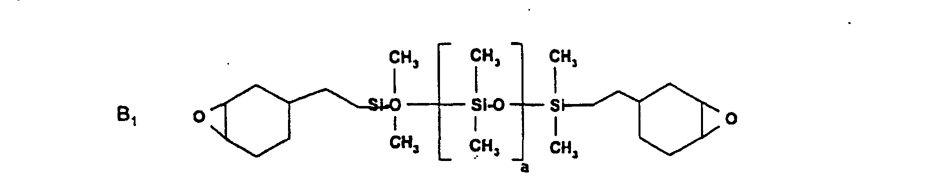 avec 89% de B₁ où a= 0 ; 9% de B₁ où a = 1 ; 0,2% de B₁ où a = 2; 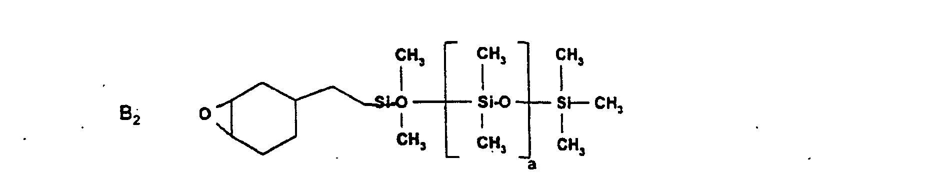 avec 0,3% de B₂ où a = 0, 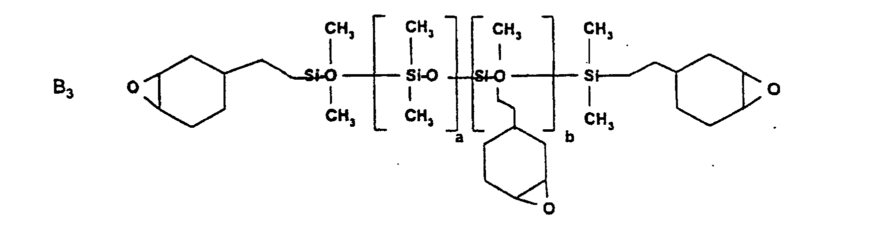 et avec 1,5% de B₃ où a = 0 et b = 1.
produit (P1): - (η⁵ - cyclopentadiènyle) (η⁶ - toluène) Fe⁺, [B(C₆F₅)₄]⁻.

### Exemple 1 - Composition pour prothèse dentaire.

On mélange à l'aide d'un agitateur tripale :
- 100 parties de siloxane **(A)** stabilisé avec 50 ppm de Tinuvin 765 ;
- 1 partie du photoamorceur **(P1)** à 75% dans l'acétate d'éthyle ;
- 150 parties d'une charge inerte à base de polyméthacrylate de méthyle pigmentée en rose (produit LUXASELF de UGL dentaire).

La composition obtenue est stable en l'absence de lumière pendant plusieurs mois à température ambiante. Cette composition peut être travaillée à la main et pendant plusieurs heures à la lumière du jour.

On réalise une éprouvette de 2,8±0.3 mm d'épaisseur dans une capsule en verre de longueur 64 mm (modèle), de largeur 10 mm (modèle) et ouverte au sommet en versant la composition préparée ("*monocomposant*") dans la capsule.

On sèche la composition en passant la capsule pendant 1 à 2 secondes (3 m/min) sous une lampe U.V. de puissance 200 W/cm correspondant à l'excitation d'un mélange de mercure et de gallium et émettant dans le domaine de l'UV visible au-delà de 400 nm.

On démoule le produit obtenu en cassant le verre.

On détermine la dureté SHORE D des deux compositions polymérisées sur chaque côté de la pièce réalisée immédiatement après la réticulation.

| Exemple 1 | Mesure immédiate | Mesure après 10 heures |
|---|---|---|
| Face irradiée: | 50 | 80 |
| Face dessous | 40 | 80 |

La dureté Shore D continue d'évoluer sensiblement pendant quelques heures.

Le retrait volumique est très faible et on obtient une excellente stabilité dimensionnelle.

La perte de masse est inférieure à 1%.

Le produit peut-être utilisé avec ou sans primaire d'accrochage en présence de dents artificielles ou de dents naturelles.

Plus généralement, les propriétés du matériau obtenu sont en accord avec la norme DIN/ISO 1567.

### Exemple 2 - Composition dentaire.

Cette composition est formulée avec :
- 95 parties de silicone **(B),**
- 0,5 partie de photoamorceur **(P1)** à 10% dans le siloxane **(B),**
- 5 parties de l'oxétane 3-éthyl-3(hydroxymethyl)-oxetane,
- et 120 parties de silice de précipitation (quartz broyé).

L'opération de réticulation-polymérisation est effectuée à l'aide d'une lampe émettant un spot lumineux émis au travers d'un embout lumineux courbe de 8 mm de diamètre. La source est une lampe Optibulb 80 W (DEMETRON Optilux 500) pour des longueurs d'ondes comprises entre 400 et 520 nm.

La composition dentaire est appliquée dans une dent. On réticule une épaisseur de 5 mm en moins de 30 secondes.

Les valeurs de rigidité trouvées sont supérieures à 60 Mpa selon la norme ISO 1567.

### Exemple 3 - composition pour prothèse dentaire ou matériau de restauration dentaire.

On mélange à l'aide d'un agitateur tripale :
- 100 parties de silicone **(B),**
- 0,5 partie de photoamorceur **(P1)** à 10% en solution dans ce silicone **(B),**
- et 120 parties de silice de précipitation (quartz broyé),

On obtient un mélange opaque de couleur grisée ne s'écoulant et manipulable.

L'opération de réticulation est effectuée de façon identique à celle de l'exemple 2. Une composition de 5 mm d'épaisseur est réticulée en moins de 30 secondes. La couleur du matériau après réticulation se rapproche de la couleur ivoire .

La composition, dans ce cas, convient notamment pour les prothèses dentaire, en particulier la rigidité est supérieure à 60 Mpa selon la norme ISO 1567.

## Revendications

1. Composition dentaire comprenant :
**(1)** au moins un oligomère ou polymère silicone réticulable et/ou polymérisable, liquide à température ambiante ou thermofusible à température inférieure à 100°C, et comprenant :
• au moins un motif de formule **(I)** : 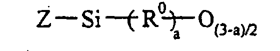
dans laquelle :
- a.= 0, 1 ou 2,
- R⁰ , identique ou différent, représente un radical alkyle, cycloalkyle, aryle, vinyle, hydrogéno, alcoxy, de préférence un alkyle inférieur en C₁-C₆,
- Z, identique ou différent, est un substituant organique comportant au moins une fonction réactive époxy, et/ou alcénylether et/ou oxétane et/ou dioxolane et/ou carbonate,
• et au moins deux atomes de silicium ;
**(2)** au moins une charge dentaire présente dans une proportion d'au moins 10% en poids par rapport au poids total de la composition ;
**(3)** et une quantité efficace d'au moins un photoamorceur de type borate de complexe organométallique, ayant une absorption résiduelle de la lumière comprise entre 200 et 500 nm ; le photoamorceur étant choisi parmi ceux de formule :
Δ dont l'entité cationique du borate est sélectionnée parmi les sels organométalliques de formule **(II)** (L¹L²L³M)^{+q} dans laquelle :
• M représente un métal du groupe 4 à 10, notamment du fer, manganèse, chrome, cobalt,
• L¹ représente 1 ligand lié au métal M par des électrons π, ligand choisi parmi les ligands η³-alkyl, η⁵⁻ cyclopendadiènyi et η⁷⁻ cycloheptratriènyl et les composés η⁶ - aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons π ;
• L² représente un ligand lié au métal M par des électrons π, ligand choisi parmi les ligands η⁷-cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π ;
• L³ représente de 0 à 3 ligands identiques ou différents liés au métal M par des électrons σ, ligand(s) choisi(s) parmi CO et NO₂⁺ ; la charge électronique totale q du complexe à laquelle contribuent L¹, L² et L³ et la charge ionique du métal M étant positive et égale à 1 ou 2 ;
Δ et dont l'entité anionique borate a pour formule [BXₐ R_{b}]⁻ **(III)** dans laquelle
- a et b sont des nombres entiers allant pour a de 0 à 3 et pour b de 1 à 4 avec a + b = 4,
- les symboles X représentent :
* un atome d'halogène (chlore, fluor) avec a = 0 à 3,
* une fonction OH avec a = 0 à 2,
- les symboles R sont identiques ou différents et représentent :
▷ un radical phényle substitué par au moins un groupement électroattracteur tel que par exemple OCF₃; CF₃, NO₂, CN, et/ou par au moins 2 atomes d'halogène (fluor tout particulièrement), et ce lorsque l'entité cationique est un onium d'un élément des groupes 15 à 17,
▷ un radical phényle substitué par au moins un élément ou un groupement électroattracteur notamment atome d'halogène dont le fluor en particulier, CF₃, OCF₃, NO₂, CN, et ce lorsque l'entité cationique est un complexe organométallique d'un élément des groupes 4 à 10,
▷ un radical aryle contenant au moins deux noyaux aromatiques tel que par exemple biphényle, naphtyle, éventuellement substitué par au moins un élément ou un groupement électroattracteur, notamment un atome d'halogène (fluor tout particulièrement), OCF₃, CF₃, NO₂, CN, quelle que soit l'entité cationique.

2. Composition selon la revendication 1 **caractérisée en ce que** le photoamorceur est choisi parmi le groupe constitué par :
- (η⁵ - cyclopentadiènyle) (η⁶ - toluène) Fe⁺, [B(C₆F₅)₄]⁻,
- (η⁵ - cyclopentadiènyle) (η⁶ - méthyl1-naphtalène) Fe⁺, [B(C₆F₅)₄]⁻,
- (η⁵ - cyclopentadiènyle) (η⁶ - cumène) Fe⁺, [B(C₆F₅)₄], et leur mélange.

3. Composition selon l'une quelconque des revendications 1 et 2 **caractérisée en ce que** Z est un substituant organique Z1 comportant au moins une fonction réactive époxy, et/ou dioxolane, et de préférence au moins une fonction réactive époxy.

4. Composition selon la revendication 3 **caractérisée en ce que** l'oligomère ou polymère polymère (1) comporte en outre d'autres fonctions réactives Z telles que les fonctions réactives Z2 alcénylether, oxétane et/ou carbonate.

5. Composition selon l'une quelconque des revendications précédentes, **caractérisée en ce que** la ou les fonctions réactives de Z sont choisies parmi les radicaux suivants : 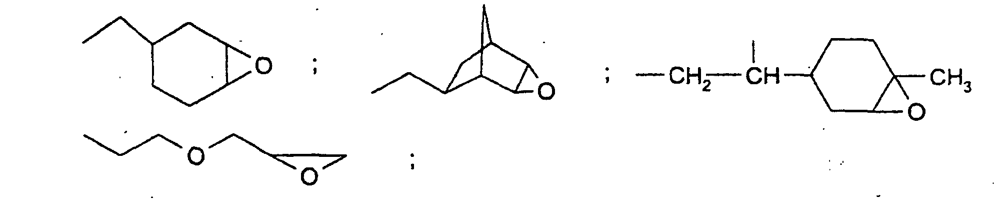 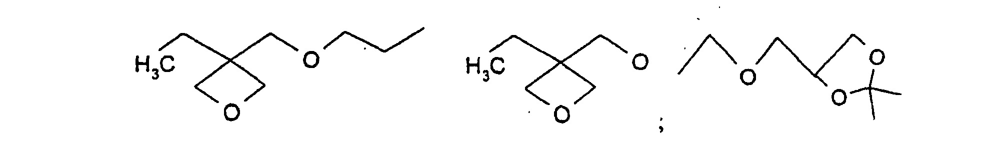
―(CH₂)₃―O―CH=CH₂ ;
―(CH₂)₃―O―CH=CH― R"
- avec R" représentant un radical alkyle linéaire ou ramifié en C₁-C₆.

6. Composition selon l'une quelconque des revendications précédentes **caractérisée en ce que** la composition dentaire comprend au moins un photosensibilisateur hydrocarboné aromatique à un ou plusieurs noyaux aromatiques substitués ou non, ayant une absorption résiduelle de la lumière comprise entre 200 et 500 nm.

7. Composition dentaire selon l'une quelconque des revendications précédentes **caractérisée en ce que** l'oligomère et/ou polymère silicone est constituée par au moins un polysiloxane de formule moyenne suivante :

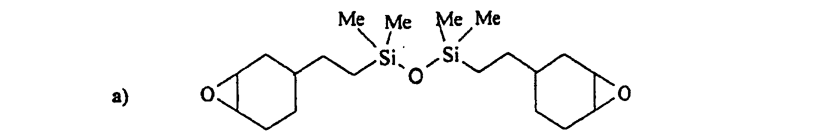

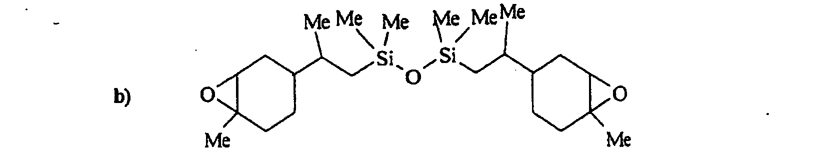

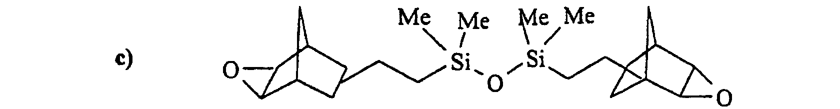

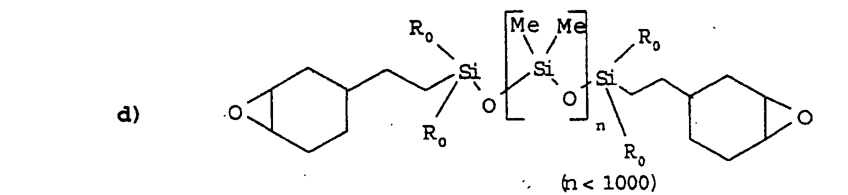

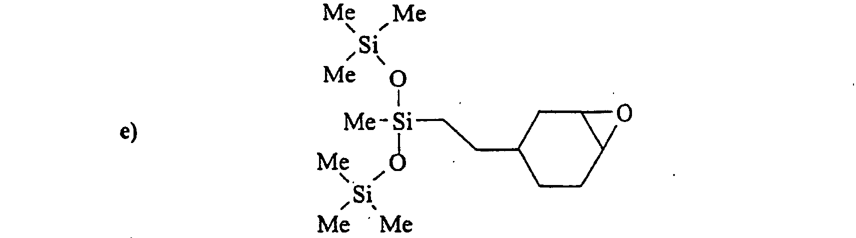

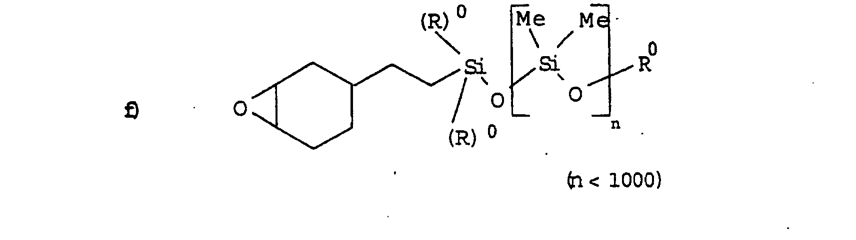

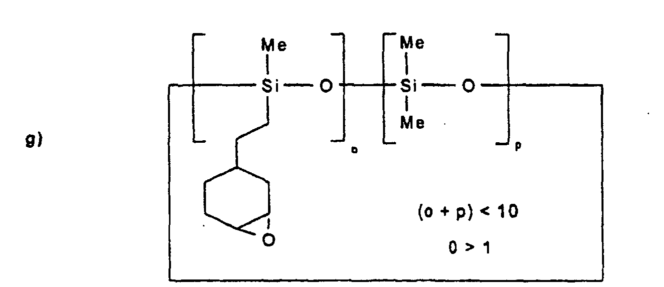

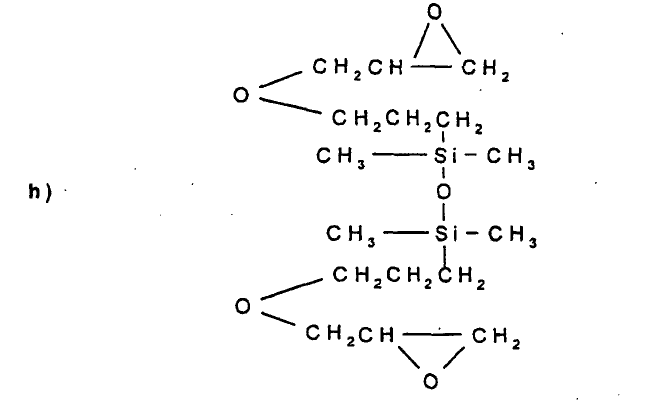

8. Utilisation d'une composition dentaire selon l'une quelconque des revendications précédentes pour la réalisation de prothèses dentaires.

9. Utilisation d'une composition dentaire selon l'une quelconque des revendications 1 à 7 pour la préparation d'une composition destinée à la restauration dentaire.

10. Prothèse dentaire susceptible d'être obtenue à partir d'une composition selon l'une quelconque des revendications 1 à 7.

11. Matériau de restauration dentaire susceptible d'être obtenu à partir d'une composition selon l'une quelconque des revendications 1 à 7.

## Patentansprüche

1. Dental-Zusammensetzung, umfassend
(1) mindestens ein vernetzbares und/oder polymerisierbares Silicon-Oligomer oder Silicon-Polymer, das bei Umgebungstemperatur flüssig oder bei Temperaturen von unter 100 °C in der Wärme schmelzbar ist, und das umfaßt:
• mindestens eine Struktureinheit der Formel (I) 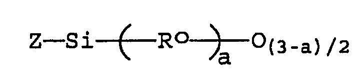 in der
- a = 0, 1 oder 2 ist,
- R°, gleich oder verschieden, einen Rest Alkyl, Cycloalkyl, Aryl, Vinyl, Hydrogeno, Alkoxy darstellt, vorzugsweise ein niederes Alkyl mit 1 bis 6 Kohlenstoffatomen,
- Z, gleich oder verschieden, ein organischer Substituent ist, umfassend mindestens eine reaktive Funktion Epoxy und/oder Alkenylether und/oder Oxetan und/oder Dioxolan und/oder Carbonat, und
• mindestens zwei Siliciumatome;
(2) mindestens einen Dentalfüllstoff, der in einem Verhältnis von mindestens 10 Gew.-% anwesend ist, bezogen auf das Gesamtgewicht der Zusammensetzung; und
(3) eine wirksame Menge von mindestens einem Photoinitiator vom Typ Borat-Organometall-Komplex mit einer Restabsorption von Licht zwischen 200 und 500 nm; wobei der Photoinitiator ausgewählt wird unter denen der Formel :
A dessen kationische Einheit von Borat selektiert wird unter den Organometall-Salzen der Formel (II) (L¹L²L³M)^{+q}, in der
• M ein Metall der Gruppe 4 bis 10 darstellt, insbesondere Eisen, Mangan, Chrom, Cobalt,
• L¹ einen Liganden darstellt, gebunden an das Metall M durch π-Elektronen, wobei der Ligand ausgewählt wird unter den Liganden η³-Alkyl, η⁵-Cyclopentadienyl und η⁷-Cycloheptatrienyl und den η⁶-aromatischen Verbindungen, ausgewählt unter den Liganden η⁶-Benzol, gegebenenfalls substituiert, und den Verbindungen mit 2 bis 4 kondensierten Ringen, wobei jeder Ring fähig ist, zur Abdeckung der Valenz des Metalls M durch 3 bis 8 π-Elektronen beizutragen;
• L² einen Liganden darstellt, gebunden an das Metall M durch n-Elektronen, wobei der Ligand ausgewählt wird unter den Liganden η⁷-Cycloheptatrienyl und den η⁶-aromatischen Verbindungen, ausgewählt unter den Liganden η⁶-Benzol, gegebenenfalls substituiert, und den Verbindungen mit 2 bis 4 kondensierten Ringen, wobei jeder Ring fähig ist, zur Abdeckung der Valenz des Metalls M durch 6 oder 7 π-Elektronen beizutragen;
• L³ 0 bis 3 gleiche oder verschiedenen Liganden darstellt, gebunden an das Metall M durch σ-Elektronen, wobei der (die) Ligand(en) ausgewählt wird (werden) unter CO und NO₂⁺; und wobei die gesamte elektronische Ladung q des Komplexes, zu der L¹, L² und L³ und die ionische Ladung des Metalls M beitragen, positiv und gleich 1 oder 2 ist; und
Δ dessen anionische Einheit Borat die Formel (III) [BXₐR_{b}]⁻ besitzt, in der:
- a und b ganze Zahlen sind, die für a von 0 bis 3 und für b von 1 bis 4 gehen, mit a + b = 4;
- die Symbole X darstellen:
* ein Halogenatom (Chlor, Fluor) mit a = 0 bis 3;
* eine Funktion OH mit a = 0 bis 2,
- die Symbole R gleich oder verschieden sind und darstellen:
> einen Rest Phenyl, substituiert durch mindestens eine elektroattraktive Gruppe, wie beispielsweise OCF₃, CF₃, NO₂, CN und/oder durch mindestens zwei Halogenatome (ganz besonders Fluor), und dies, wenn die kationische Einheit ein Onium eines Elementes der Gruppen 15 bis 17 ist,
> einen Rest Phenyl, substituiert durch mindestens ein Element oder eine elektroattraktive Gruppe, insbesondere ein Halogenatom (ganz besonders Fluor), CF₃, OCF₃, NO₂, CN, und dies, wenn die kationische Einheit ein Organometallkomplex eines Elementes der Gruppen 4 bis 10 ist,
> einen Rest Aryl, enthaltend mindestens zwei aromatische Kerne, wie beispielsweise Biphenyl, Naphthyl, gegebenenfalls substituiert durch mindestens ein Element oder eine elektroattraktive Gruppe, insbesondere ein Halogenatom (ganz besonders Fluor), OCF₃, CF₃, NO₂, CN, wie die kationische Einheit auch sein mag.

2. Zusammensetzung nach Anspruch 1, **dadurch gekennzeichnet, daß** der Photoinitiator aus der Gruppe gewählt wird, die gebildet wird durch:
- (η⁵-Cyclopentadienyl) (η⁶-toluol) Fe⁺, [B(C₆F₅)₄]⁻ ,
- (η⁵-Cyclopentadienyl) (η⁶-1-methylnaphthalin)Fe⁺, [B(C₆F₅)₄]⁻ ,
- (η⁵-Cyclopentadienyl) (η⁶-cumol)Fe⁺, [B(C₆F₅)₄]⁻,
- und ihre Mischungen.

3. Zusammensetzung nach irgendeinem der Ansprüche 1 und 2, **dadurch gekennzeichnet, daß** Z ein organischer Substituent Z1 ist, der mindestens eine reaktive Funktion Epoxy und/oder Dioxolan und vorzugsweise mindestens eine reaktive Funktion Epoxy umfaßt.

4. Zusammensetzung nach Anspruch 3, **dadurch gekennzeichnet, daß** das Oligomer oder Polymer (1) außerdem andere reaktive Funktionen Z umfaßt wie die reaktiven Funktionen Z2 Alkenylether, Oxetan und/oder Carbonat.

5. Zusammensetzung nach irgendeinem der vorstehenden Ansprüche, **dadurch gekennzeichnet, daß** die reaktive(n) Funktion(en)Z ausgewählt werden unter den folgenden Resten: 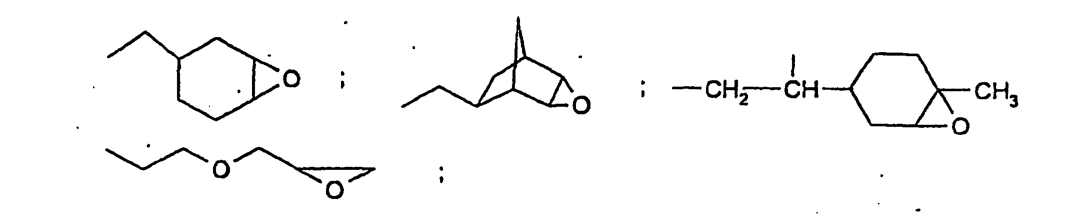 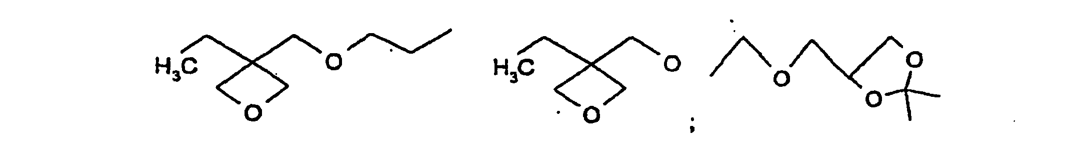
―(CH₂)₃―O―CH=CH₂ ;
―(CH₂)₃―O―CH=CH― R"
- worin R" einen linearen oder verzweigten Rest Alkyl mit 1 bis 6.Kohlenstoffatomen darstellt.

6. Zusammensetzung nach irgendeinem der vorstehenden Ansprüche, **dadurch gekennzeichnet, daß** die Dental-Zusammensetzung mindestens einen aromatischen kohlenwasserstoffartigen, substituierten oder unsubstituierten Photosensibilisator mit einem oder mehreren aromatischen Kernen und einer Restabsorption von Licht zwischen 200 und 500 nm umfaßt.

7. Dental-Zusammensetzung nach irgendeinem der vorstehenden Ansprüche, **dadurch gekennzeichnet, daß** das Silicon-Oligomer oder Silicon-Polymer aus mindestens einem Polysiloxan der folgenden mittleren Formeln gebildet wird:

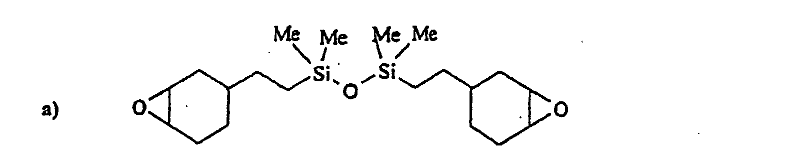

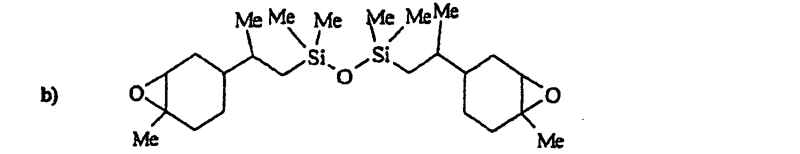

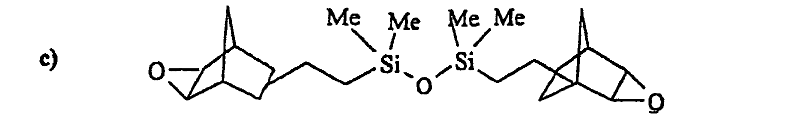

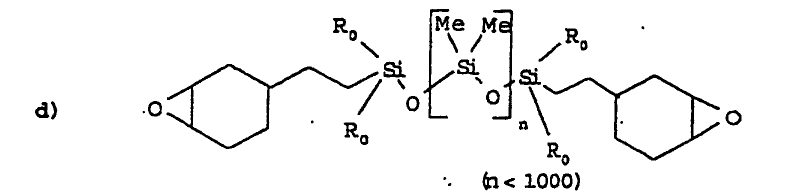

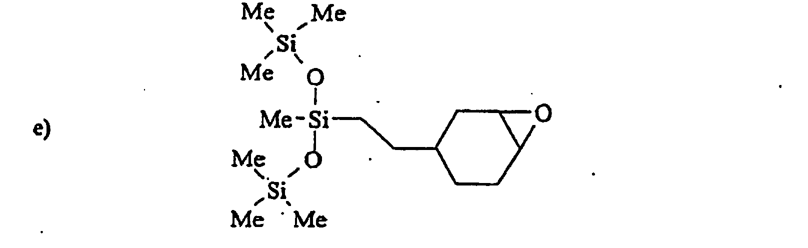

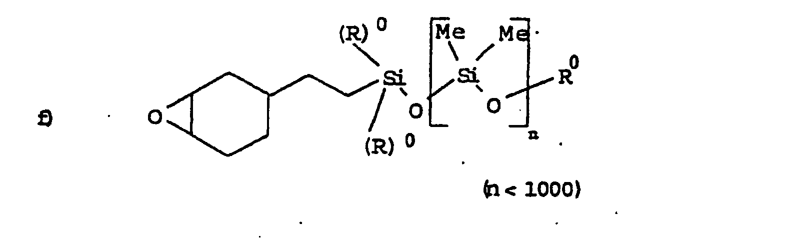

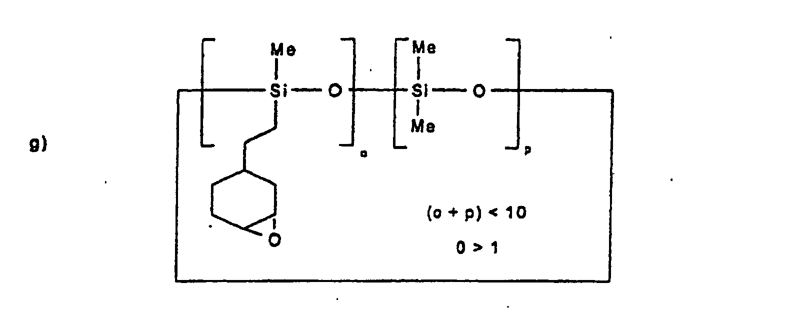

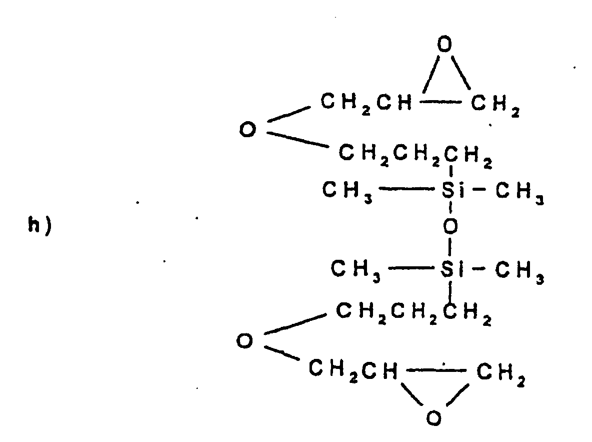

8. Verwendung einer Dental-Zusammensetzung nach irgendeinem der vorstehenden Ansprüche für die Herstellung von Zahnprothesen.

9. Verwendung einer Dental-Zusammensetzung nach irgendeinem der Ansprüche 1 bis 7 für die Herstellung einer Zusammensetzung, die für die Zahn-Restaurierung vorgesehen ist.

10. Zahnprothese, die geeignet ist, ausgehend von einer Zusammensetzung nach irgendeinem der Ansprüche 1 bis 7 erhalten zu werden.

11. Material zur Zahn-Restaurierung, das geeignet ist, ausgehend von einer Zusammensetzung nach irgendeinem der Ansprüche 1 bis 7 erhalten zu werden.

## Claims

1. Dental composition comprising:
**(1)** at least one crosslinkable and/or polymerizable silicone oligomer or polymer which is liquid at room temperature or which is heat-meltable at a temperature of less than 100°C, and which comprises:
• at least one unit of formula (I) : 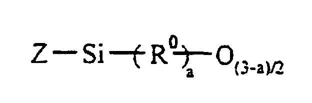 in which:
- a = 0, 1 or 2,
- R⁰, identical or different, represents an alkyl, cycloalkyl, aryl, vinyl, hydrogeno or alkoxy radical, preferably a C₁-C₆ lower alkyl,
- Z, identical or different, is an organic substituent comprising at least one reactive epoxy, and/or alkenyl ether and/or oxetane and/or dioxolane and/or carbonate functional group,
• and at least two silicon atoms;
**(2)** at least one dental filler present in a proportion of at least 10% by weight relative to the total weight of the composition;
**(3)** and an effective quantity of at least one organometallic complex borate-type photoinitiator having a residual light absorption of between 200 and 500 nm, the photoinitiator being chosen from those of formula:
Δ in which the cationic entity of the borate is selected from the organometallic salts of formula (II) (L¹L²L³M)^{+q}, in which:
• M represents a group 4 to 10 metal, in particular iron, manganese, chromium or cobalt;
• L¹ represents 1 ligand bound to the metal M by π electrons, which ligand is chosen from the ligands η³-alkyl, η⁵-cyclopentadienyl and η⁷-cycloheptatrienyl and the η⁶-aromatic compounds chosen from the optionally substituted η⁶-benzene ligands and the compounds having from 2 to 4 condensed rings, each ring being capable of contributing to the valency layer of the metal M by 3 to 8 π electrons;
• L² represents a ligand bound to the metal M by π electrons, which ligand is chosen from the ligands η⁷-cycloheptatrienyl and the η⁶-aromatic compounds chosen from the optionally substituted ligands η⁶-benzene and the compounds having from 2 to 4 condensed rings, each ring being capable of contributing to the valency layer of the metal M by 6 or 7 π electrons;
• L³ represents from 0 to 3 ligands, which are identical or different, linked to the metal M by σ electrons, which ligand(s) is (are) chosen from CO and NO₂⁺; the total electron charge q of the complex to which L¹, L² and L³ contribute and the ionic charge of the metal M being positive and equal to 1 or 2;
Δ and the anionic borate entity of which has the formula [BXₐR_{b}]⁻ (III) in which:
- a and b are integers ranging, for a, from 0 to 3 and, for b, from 1 to 4 with a + b = 4,
- the symbols X represent:
* a halogen atom (chlorine, fluorine) with a = 0 to 3,
* an OH functional group with a = 0 to 2,
- the symbols R are identical or different and represent:
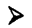 a phenyl radical substituted with at least one electron-attracting group such as for example OCF₃, CF₃, NO₂, CN, and/or with at least 2 halogen atoms (fluorine most particularly), this being when the cationic entity is an onium of an element of groups 15 to 17,
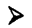 a phenyl radical substituted with at least one element or one electron-attracting group, in particular a halogen atom, including fluorine in particular, CF₃, OCF₃, NO₂, CN, this being when the cationic entity is an organometallic complex of an element of groups 4 to 10,
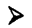 an aryl radical containing at least two aromatic nuclei such as for example biphenyl, naphthyl, optionally substituted with at least one electron-attracting group or element, in particular a
halogen atom (fluorine most particularly), OCF₃, CF₃, NO₂, CN, regardless of the cationic entity.

2. Composition according to Claim 1, **characterized in that** the photoinitiator is chosen from the group consisting of:
- (η⁵-cyclopentadienyl) (η⁶-toluene) Fe⁺, [B(C₆F₅)₄]⁻,
- (η⁵-cyclopentadienyl) (η⁶-methyl-1-naphthalene) Fe⁺, [B(C₆F₅)₄]⁻,
- (η⁵-cyclopentadienyl) (η⁶-cumene) Fe⁺, [B(C₆F₅)₄]⁻, and the mixture thereof.

3. Composition according to either of Claims 1 and 2, **characterized in that** Z is an organic substituent Z1 comprising at least one reactive epoxy, and/or dioxolane functional group, and preferably at least one reactive epoxy functional group.

4. Composition according to Claim 3, **characterized in that** the oligomer or polymer (1) comprises, in addition to other reactive functional groups Z, reactive functional groups Z2 such as alkenyl ether, oxetane and/or carbonate.

5. Composition according to any one of the preceding claims, **characterized in that** the reactive functional group(s) of Z are chosen from the following radicals: 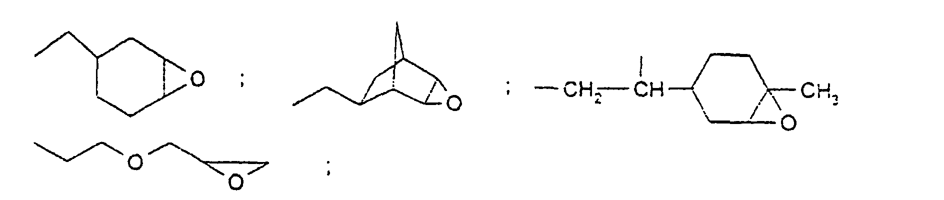 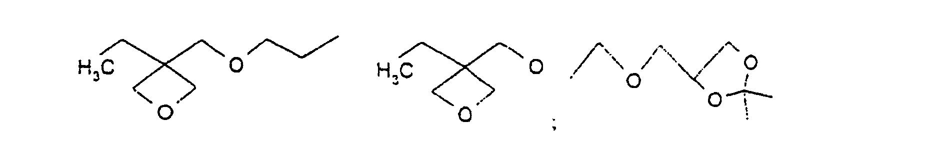
―(CH₂)₃―O―CH=CH₂ ;
―(CH₂)₃―O―CH=CH― R"
- with R" representing a linear or branched C₁-C₆ alkyl radical.

6. Composition according to any one of the preceding claims, **characterized in that** the dental composition comprises at least one aromatic hydrocarbon photosensitizer with one or more aromatic nuclei which are substituted or not, having a residual light absorption of between 200 and 500 nm.

7. Dental composition according to any one of the preceding claims, **characterized in that** the silicone oligomer and/or polymer consists of at least one polysiloxane having the following average formula:

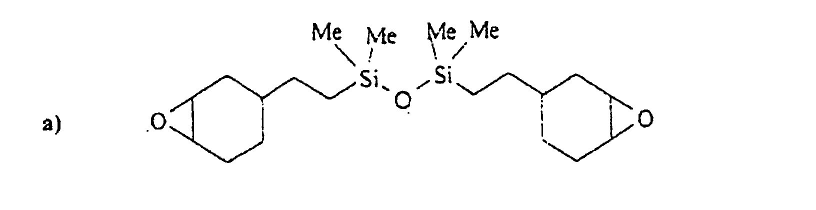

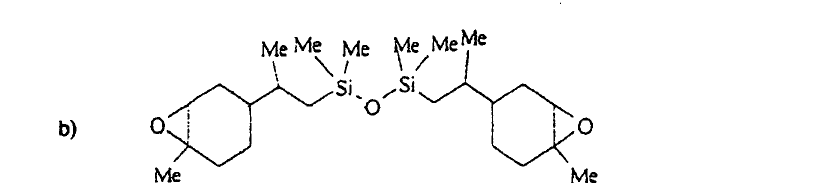

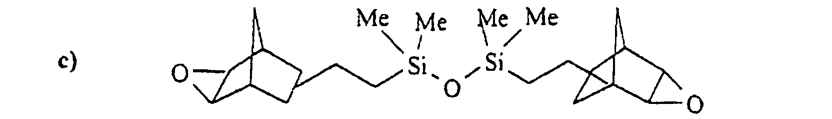

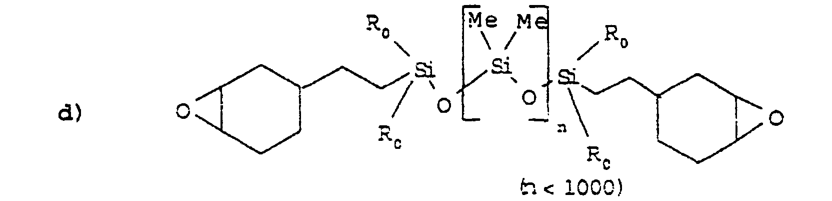

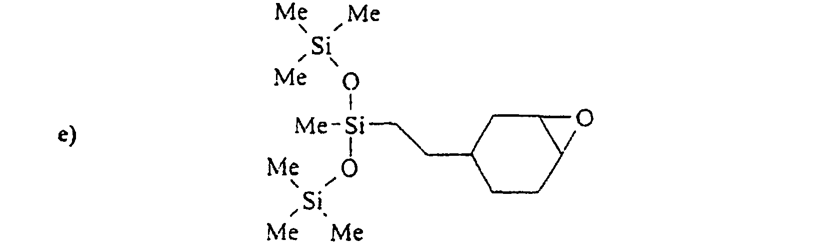

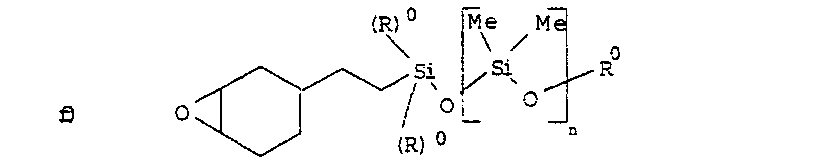

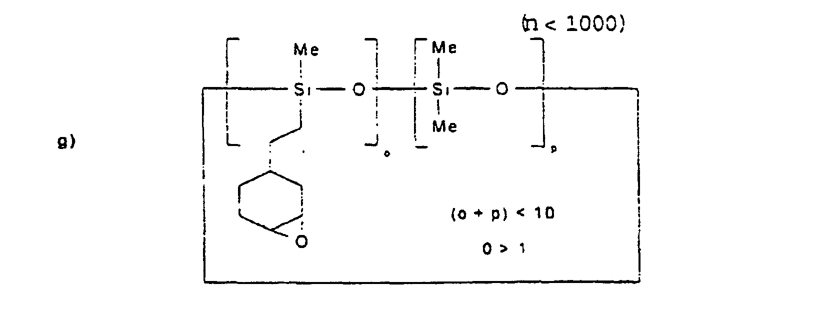

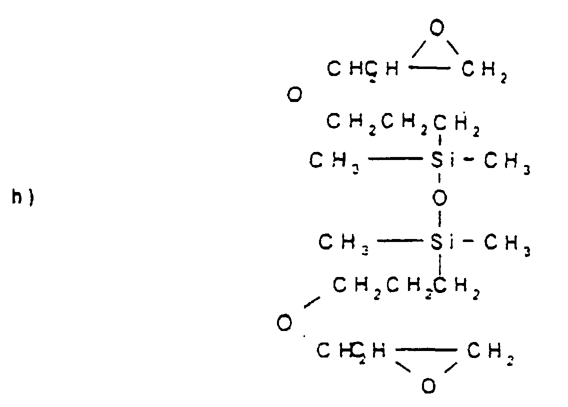

8. Use of a dental composition according to any one of the preceding claims for the production of dental prostheses.

9. Use of a dental composition according to any one of Claims 1 to 7 for the preparation of a composition intended for dental restoration.

10. Dental prosthesis which can be obtained from a composition according to any one of Claims 1 to 7.

11. Dental restoration material which can be obtained from a composition according to any one of Claims 1 to 7.
